(19) Europäisches Patentamt
European Patent Office
Office européen des brevets

(11) Numéro de publication: **0 001 733**
**B1**

(12) **FASCICULE DE BREVET EUROPEEN**

(45) Date de publication du fascicule du brevet: **23.06.82**

(21) Numéro de dépôt: **78400141.4**

(22) Date de dépôt: **20.10.78**

(51) Int. Cl.³: **C 07 D 493/04**, A 61 K 31/37 //C07C43/20, C07D307/79, C07D307/83, (C07D493/04, 311/00, 307/00)

# (54) 5-Méthoxy-psoralène comme nouveau médicament et procédé de synthèse.

(30) Priorité: **21.10.77 FR 7731719**
**09.12.77 FR 7737144**

(43) Date de publication de la demande:
**02.05.79 Bulletin 79/9**

(45) Mention de la délivrance du brevet:
**23.06.82 Bulletin 82/25**

(84) Etats contractants désignés:
**CH DE GB NL SE**

(56) Documents cités:
**CHEMICAL ABSTRACTS, vol. 65, 1966, 6077f. KHADZHAI et al. "Relation between structure and spasmolytic activity in coumarin and furocoumarin derivatives".**

**TETRAHEDRON LETTERS, 1969, 5223—4.**

**CHEMICAL ABSTRACTS, vol. 70, 1969, 57701d. CAPORALE et al. "Methylpsoralens"**

**RUSSIAN DRUG INDEX. 2é Edition. 1967. p. 111.**

**DERMATOLOGIE IN PRAXIS UND KLINIK. KORTING. BAND I (1980). p. 7. 118.**

(73) Titulaire: **Goupil, Jean-Jacques**
**30 Avenue du Président Wilson**
**F-94230 CACHAN (FR)**

(72) Inventeur: **Goupil, Jean-Jacques**
**30 Avenue du Président Wilson**
**F-94230 CACHAN (FR)**

(74) Mandataire: **Petit, Hélène**
**CABINET Pierre LOYER 18 Rue de Mogador**
**F-75009 PARIS (FR)**

**EP 0 001 733 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

5-méthoxy-psoralène comme nouveau médicament et procédé de synthèse

La présente invention a pour objet un nouveau médicament caractérisé en ce qu'il contient comme substance active le 5-méthoxy-psoralène de formule:

On sait que certaines furocoumarines dont le psoralène et certains de ses dérivés, présente une activité photodynamique génératrice de photodermites. Ces dernières apparaissent après administration orale ou topique desdites furocoumarines et exposition ou soleil ou aux rayons ultra-violets.

Dans les études qui ont été faites sur la phototoxicité du psoralène et de ses dérivés, il a été démontré que le plus photodynamique ou phototoxique était le psoralène lui-même de formule:

venaient ensuite par ordre décroissant le 4'-méthyl-psoralène, le 8-méthoxy-psoralène, la 4,5'-diméthylxanthotoxine, la 4-méthylxanthotoxine, le 5-méthoxy-psoralène, le 5-éthoxy-psoralène. La décroissance de la phototoxicité était importante puisque pour le 8-méthoxy-psoralène, elle n'était plus que de 37,5% par rapport au psoralène et que pour le 5-méthoxy-psoralène elle était de 27,5%. On a également trouvé que le 8-méthoxy-psoralène était utile dans le traitement du psoriasis. Les résultats obtenus sont bons en ce qu'on obtient un "blanchiment" important du psoriasis. Les effets secondaires observés sont l'apparition d'érythèmes, de nausées, de prurit, de maux de tête, qui dérivent de la toxicité du produit et de celle de l'irradiation intense.

Selon la présente invention, on a découvert que contrairement à ce que l'on pouvait penser, un autre dérivé du psoralène, nettement moins phototoxique, c'est-à-dire moins actif, présentait néanmoins un ensemble de propriétes qui le rendaient de manière tout à fait inattendue beaucoup plus apte, à un usage thérapeutique et plus efficace dans le traitement du psoriasis.

Ce dérivé est le 5-méthoxy-psoralène. Il peut être extrait de l'essence de bergamote naturelle ou synthétisé par la nouvelle méthode de préparation donnée ci-après.

Il s'agit selon la présente invention d'une nouvelle synthèse du 5-méthoxy-psoralène de formule:

à partir du phloroglucinol de formule:

Le phloroglucinol est choisi comme matière première en raison de la présence de trois groupements hydroxylés sur son noyau.

Dans une première étape, on procède à une méthylation par le méthanol en présence d'un courant

d'acide chlorhydrique gazeux et on obtient un mélange d'éther mono et diméthylique du phloroglucinol, mélange dont on extrait par distillation fractionnée l'éther monométhylique de formule:

On a ainsi, dès la première étape, placé le substituant méthoxy du 5-méthoxy-psoralène dans la position désirée.

Dans une seconde étape, on réalise la cyclisation de l'éther monométhylique de phloroglucinol pour obtenir l'hydroxy-6 méthoxy-4 coumaranone-3 de formule:

Une première réaction avec du chloro-acétonitrile, de l'acide chlorhydrique gazeux et du chlorure de zinc conduit à un mélange de la coumaranone cherchée et de chloro-acéto-4méthoxy-5 résorcinol de formule:

Ce dernier corps est à son tour cyclisé en coumaranone en soumettant ce mélange à un chauffage à reflux avec de l'acétate de potassium.

Dans une troisième étape, on procède à la conversion de l'hydroxy-6 méthoxy-4 coumarane-3 one en hydroxy-6 méthoxy-4-coumarane par réduction directe avec de l'hydrate d'hydrazine comme catalyseur.

$\xrightarrow[(KOH)]{H_2N{-}NH_2}$

Cette réduction directe de l'hydroxy-6-méthoxy-4-coumaranone est tout à fait nouvelle. Jusqu'à présent les méthodes de réduction utilisables comprenaient plusieurs étapes. En particulier la réduction avec un catalyseur au carbone palladié nécessitait la protection préalable du groupement hydroxylé par une réaction d'acétylation par le chlorure d'acétyle, puis après réduction une opération de désacétylation pour libérer le groupement hydroxyle et pouvoir poursuivre les réactions de cyclisation.

Selon la présente invention, on procède à la réduction directe en coumarane, en dissolvant à chaud la coumarane-3 one dans du diéthylène glycol, en ajoutant de l'hydrate d'hydrazine à 98%, en chauffant à reflux pendant 15 mn avec formation de l'hydrazone correspondante, en ajoutant après refroidissement de la potasse en pastilles et en portant à douce ébullition pendant 10 heures. Après acidification jusqu'à pH 27,5, extraction à chaud à l'éther, en contre courant liquide-liquide, distillation et recristallisation, on obtient de fins cristaux jaune clair de coumarane avec un rendement de 70%.

Cette réaction de réduction directe de l'hydroxy-méthoxy-coumaranone-one est possible grâce au choix de l'hydrate d'hydrazine comme catalyseur de réduction. Ce dernier opère sélectivement et ne

réagit pas sur le groupement hydroxylé. Les avantages de ce nouveau procédé sont nombreux: suppression de deux étapes au moins acétylation et désacétylation, choix d'un catalyseur de bonne capacité de réduction et meilleur marché que les catalyseurs au palladium, absence des problèmes de vieillisement et de régénération du catalyseur.

Dans une quatrième étape, on procède à la cyclisation de l'hydroxy-6 méthoxy-4 coumarane en tétrahydro-3, 4, 4', 5'-méthoxy-5 psoralène avec de l'acrylonitrile, du chlorure de zinc et un courant d'acide chlorhydrique gazeux.

$OCH_3$ ... OH —[$CH_2=CH-CN$ / $ZnCl_2$ HCl]→ $OCH_3$ ... O O

Dans une cinquième étape il ne reste plus qu'à déshydrogéner le composé précédent, soit sur charbon palladié, soit avec du chloranile, soit par tout autre agent déshydrogénant pour obtenir le 5-méthoxy-psoralène de formule:

$OCH_3$ ... O O O

Ce produit obtenu en fines paillettes nacrées incolores de PF = 188°C, présente des spectres IR, UV, RMN identiques à ceux du produit naturel.

Le mode opératoire détaillé de cette nouvelle synthèse est donné ci-après:

Ier Stade: éther mono-méthylique du phloroglucinol.

Dans un ballon muni d'un réfrigérant ascendant et d'un tube plongeant jusqu'au fond du mélange réactionnel, on introduit:

— 40 g de phloroglucinol anhydre;

— 200 ml de méthanol (absolu).

On porte au reflux tout en passant un lent courant d'HCl gazeux, pendant 3 heures pour maintenir la saturation (environ: 15 g d'HCl gazeux sont absorbés). On laisse au repos pendant une nuit.

On chasse l'excès de méthanol au bain-marie sous léger vide (cet alcool récupéré peut servir pour une nouvelle méthylation).

Le résidu sirupeux est repris par 50 ml d'eau et 50 ml d'éther. Décanter et extraire encore deux autres fois, avec chaque fois 50 ml d'éther.

Les solutions éthérées sont réunies, lavées à l'eau, puis avec une solution de $CO_3NaH$ à 5% jusqu'à neutralité, enfin une dernière fois à l'eau.

On sèche sur du chlorure de calciumaanhydre;

On filtre, chasse l'excès d'éther au bain-marie;

On rectifie le résidu de la chasse sous pression réduite.

Résultats:

a) dans les fractions de tête $E_{15}$ 180°—195° on a le di-éther méthylique;

b) dans les fractions de coeur: $E_{15}$ 195—210° on a un mélange des deux éthers.

On triture à froid dans du toluène qui dissout le di-éther.

On essore, lave au toluène; on obtient des cristaux incolores d'éther monométhylique de PF = 79°C

$C_7H_8O_3$ Poids moléculaire: 140

rendement: 55%

Le contrôle analytique a donné les résultats suivants:

| | calc. | trouvé. |
|---|---|---|
| Carbone % | 60,05 | 59,98 |
| Hydrogène % | 05,76 | 05,80 |
| Oxygène % | 34,29 | 34,30 |

Réaction:

OH

$CH_3O$ $OCH_3$

Ether diméthylique

F = 37°C

OH

HO OH

CH OH,HCl Gazeux

$OCH_3$

HO OH

Ether mono-méthylique

F = 79°C

2ème Stade: Hydroxy-6 méthoxy-4 coumaranone-3
Réaction:

OCH3
HO
OH
Cl–CH2–CN, Zn Cl2
HCl gazeux, CH3–COOK

$OCH_3$ O 3 4 6 O OH

Dans une suspension bien refroidie (et vigoureusement agitée) de:

— 40 ml d'éther anhydre ou de ligroïne (Eb 40°—60°C)
— 7,70 g (ou 0,055 mole) d'éther mono-méthylique de phloroglucinol,
— 4 g (ou 0,053 mole) de chloro-acétonitrile fraîchement rectifié,
— 4 g (ou 0,03 mole) de chlorure de zinc (fondu et pulvérisé)

on fait barboter un courant d'HCl gazeux pendant 35 mn.

Le chlorhydrate de cétimine est essoré, lavé deux fois avec 10 ml d'éther anhydre. On le dissout dans 20 ml d'eau distillée (les dernières traces d'éther accompagnant la solution aqueuse sont éliminées par aspiration à la trompe à eau).

Cette solution aqueuse est chauffée au reflux pendant 10 mn, puis laissée au repos pendant une nuit dans un réfritérateur.

Il se forme un précipité qui est un mélange de deux produits:

1) Hydroxy-6 méthoxy-4 coumaranone-3
2) Chloro-acéto-4 méthoxy-5 résorcinol-1,3 (de couleur jaune)

$ClCH_2-CO$ — $OCH_3$ — HO — OH

On essore ce mélange et le lave deux fois, avec chaque fois 20 ml d'eau glacée. On le sèche. Poids: environ 8,30 g

Traitement du mélange pour cycliser le chloro-acéto-4 méthoxy-5 résorcinol-1,3:

On introduit ce mélange à une solution de 5 g d'acétate de potassium dans 25 ml d'éthanol absolu.

On chauffe au reflux pendant 15 mn; on refroidit, verse le mélange réactionnel dans 60 ml d'eau glacée.

On essore le précipité et le recristallise dans environ 30 ml d'eau bouillante.

En refroidissant, la coumaranone cherchée se présente sous forme de fines aiguilles jaune-clair F: 305°.

Rendement: 80% environ.

$C_9H_8O_4$ Poids moléculaire: 180

Les résultats de l'analyse centésimale sont les suivants:

| | Calc. | Trouvé. |
|---|---|---|
| Carbone % | 60,05 | 59,98 |
| Hydrogène % | 4,48 | 4,59 |
| Oxygène % | 35,56 | 35,50 |

C.C.M. Silicagel F-1500 LS-254

Solvant: chlorure de méthylène/éthanol: 30/5 Rf 0,76

Test au chlorure ferrique: positif (pour le groupement OH).

3ème Stade: hydroxy-6 méthoxy-4 coumarane

OCH3
O
3
4
6
OH
H2N–NH2 (KOH)
OCH3
4
6
O
OH

On dissout à chaud 10 g de coumarane-3 one, dans 100 ml de diéthylène glycol

$$\left[ O(CH_2CH_2OH)_2 \quad Eb \quad 244° \right]$$

On refroidit partiellement et ajoute 20 g d'hydrate d'hydrazine à 98%. On chauffe au reflux pendant 15 mn (à contrôler que toute la cétone est transformée en hydrazone par C.C.M.). On refroidit.

On ajoute 30 g de potasse en pastilles et réchauffe en douce ébullition pendant 10 heures.

On verse sur glace, acidifie jusqu'à pH~7,5 et extrait à l'éther, à chaud, en contre-courant. On lave les solutions éthérées à l'eau, sèche sur du sulfate de sodium anhydre. On filtre et chasse l'excès de solvant.

On distille sout haut vide $E_{0,2}$ 164°C et on recristallise dans un mélange hydro-alcoolique.

Fins cristaux jaune-clair F: 77°C

C.C.M. silicagel F-1500 LS-254 Rendement 70%

Solvant: — chlorure de méthylène/éthanol: 30/5
Rf~0,85
— chlorure de méthylène/acétate d'éthyle: 25/5
Rf~0,80

Le Rf de la coumaranone est toujours plus petit que celui du coumarane.
$C_9H_{10}O_3$ Poids moléculaire: 166,17
Les résultats de l'analyse centésimale sont les suivants:

| | Calc. | Trouvé. |
|---|---|---|
| Carbone % | 65,05 | 64,71—64,53 |
| Hydrogène % | 6,06 | 6,02— 5,98 |
| Azote % | 28,89 | 28,06—28,41 |

4ème stade: Tétrahydro-3, 4, 4', 5' méthoxy-5 psoralène

$CH_2 = CH{-}CN$ ; Zn $Cl_2$, HCl gazeux

Sous bonne agitation magnétique, en maintenant la température intérieure entre 0°—5°C, on fait barboter un courant d'HCl gazeux dans un mélange de:
— 150 ml d'éther anhydre . . . (séché sur sodium)
— 4,65 g (ou 0,028 mole) . . . d'hydroxy-6 méthoxy-4 coumarane
— 3,80 g (ou 0,028 mole) . . . de chlorure de zinc anhydre
— 2,12 g (ou 0,01 mole) . . . d'acrylo-nitrile (stabilisé).

Après 30 mn, une huile rouge se sépare. On continue l'agitation et l'introduction d'HCl gazeux pendant deux autres heures.

On garde la masse réactionnelle pendant 2 jours dans l'obscurité.

On décante la couche éthérée, ajoute 25 ml d'eau à l'huile et chauffe au bain-marie bouillant pendant une heure.

On refroidit, extrait plusieurs fois à l'éther, lave les solutions éthérées à l'eau froide, sèche sur du sulfate de sodium anhydre, filtre, chasse l'excès de solvant.

On peut ou sublimer ou distiller sous haut vide ou recristalliser dans l'éther de pétrole ($E_{40°-60°C}$).
Rendement: 75% fins cristaux F: 175°C
$C_{12}H_{12}O_4$ Poids moléculaire: 220,18
Les résultats de l'analyse centésimale sont les suivants:

| | Calc. | Trouvé |
|---|---|---|
| Carbone % | 65,50 | 65,45—65,70 |
| Hydrogène % | 05,49 | 05,40—05,30 |
| Azote % | 29,07 | 28,80—28,90 |

5ème stade: Déshydrogénation 1 méthoxy-5 psoralène.

charbon palladié ou chloranile

Plusieurs méthodes:

1°) — Chauffer au reflux pendant 5 heures un mélange de:

— 15 g de diphényl-oxyde (F: 28°)
— 1 g de tétrahydro-3, 4, 4', 5' méthoxy-5 psoralène
— 1 g de charbon palladié à 10% (frais).

Filtrer à chaud pour éliminer le catalyseur. Chasser ou sous bon vide d'excès d'ether diphénylique ou par entraînement à la vapeur d'eau.

Recristalliser dans l'éthanol ou dans le methanol (1 g soluble dans 70 ml d'éthanol absolu).

2°) — On peut également déshydrogéner avec le chloranile ou tetrachloro benzoquinone-1,4 ou tout autre agent déshydrogénant en utilisant comme solvant: diphényl-oxyde ou diphényl-éther-butanol-xylène ....

Spectre IR, — id— UV, — id— RMN: Conformes à ceux de la littérature et identiques à ceux du produit naturel

Chromato sous hautes pressions.

Pas d'abaissement du P.F. eutectique (du mélange produit naturel et produit synthétique).

Fines paillettes nacrées, incolores F: 188°.
photo-sensibles.

Rendement: 80%

$C_{12}H_8O_4$ Poids moléculaires: 216,18

Les resultats de l'analyse centésimale sont les suivants:

| | Calc. | Trouvé. |
|---|---|---|
| Carbone % | 66,67 | 66,65—66,85 |
| Hydrogène % | 3,73 | 3,60— 3,65 |
| Oxygène % | 29,60 | 29,46—29,48 |

Le procédé de synthèse du 5-méthoxy-psoralène selon la presente invention est particulièrement intéressant en ce qu'il part d'une matière première d'un prix convenable, qu'il présente un nombre d'étapes réduit par rapport aux synthèses connues des autres dérivés du psoralène, qu'il utilise un catalyseur de réduction de la coumaranone bon marché et sans les inconvénients de vieillisement et de régénération attachés à d'autres catalyseurs, et qu'il donne de bons rendements.

La phototoxicité du 5-méthoxy-psoralène est intérieure à celle du 8-méthoxy-psoralène d'environ 20 à 30%, d'après les études cliniques comparées qui ont été effectuées dans le cadre de l'invention par administration orale de doses de 40 mg des deux composés.

Par administration topique dans des concentrations supérieures à 100 ppm, le 5-méthoxy-psoralène est également apparu comme beaucoup moins phototoxique que le 8-méthoxy-psoralène.

L'étude comparée de la toxicité aiguë du 5-méthoxy-psoralène et du 8-méthoxy-psoralène a été effectuée sur des souris et des rats mâles, exempts d'organismes pathogènes spécifiques et soumis à une diète hydrique préalable de 18 heures.

Tous les animaux ont été gardés en observation pendant 14 jours après l'administration orale unique afin de détecter une éventuelle toxicité retardée. La dose léthale 50 et ses limites de confiance ont été calculées selon la méthode graphique de J. T. Litchfield et F. Wilcoxon.

Dans ces essais, l'administration se faisait par voie orale, l'agent dispersif était une solution aqueuse de carboxy-méthyl-cellulose à 2p. 100 additionnée de 0,5p. 100 de Tween 80. Chez les souris mâles, on a trouvé une DL 50 en 14 jours de 875 mmg/kg pour le 8-méthoxy-psoralène et de 8100 mg/kg pour le 5-méthoxy-psoralène. Chez les rats mâles, on a trouvé une DL 50 en 14 jours de 4400 mg/kg pour le 8-méthoxy-psoralène et supérieure à 30.000 mg/kg pour le 5-méthoxy-psoralène.

En conclusion, on peut considérer que le 5-méthoxy-psoralène administré par voie orale est environ 9,25 fois moins toxique que le 8-méthoxy-psoralène.

Des expériences analogues ont été faites sur le cobaye et ont montré que sur cet animalle 5-méthoxy-psoralène est environ 17 fois moins toxique que le 8-méthoxy-psoralène.

C'est la découverte inattendue de la faible toxicité aiguë du 5-méthoxy-psoralène et de l'existence d'une remarquable activité thérapeutique qui a conduit à présenter de nouvelles compositions pharmaceutiques contenant ce produit.

En effet, grâce à cette faible toxicité, on peut utiliser le 5-méthoxy-psoralène à des doses thérapeutiques très supérieures aux maxima utilisables pour le 8-méthoxy-psoralène tout en restant à des niveaux très inférieurs de toxicité.

Si l'on définit l'index thérapeutique comme le rapport entre l'activité thérapeutique (mesurée par la phototoxicité) et la toxicité aiguë et que l'on suppose que pour le 8-méthoxy-psoralène ce rapport est de 100:100, c'est-à-dire 1, pour le 5-méthoxy-psoralène il sera de 75 (diminution de 25% de l'activité thérapeutique): 6 (toxicité 17 fois moins grande), c'est-à-dire de 12,5.

On peut donc conclure que l'index thérapeutique du 5-méthoxy-psoralène est de 8 à 12 fois supérieur à celui du 8-méthoxy-psoralène.

Dans ces conditions, on peut utiliser des doses thérapeutiques de 5-méthoxy-psoralène jusqu'à 5 fois supérieures à celles utilisées pour le 8-méthoxy-psoralène, ce qui est considérable.

On a également étudié la toxicité générale à long terme du 5-méthoxy-psoralène dans des traitements de lapins par voie gastrique et par application dermique. Les résultats sont les suivants.

Pour une administration par voie gastrique pendant 42 jours consécutifs de 5-méthoxy-psoralène à la dose quotidienne de 300 mg/kg, l'étude toxicologique (état général, examen de la peau et du fond de l'oeil, examen hématologique, de chimie clinique, et anatomopathologique) démontre la bonne tolérance du nouveau médicament et ne fournit aucun élément susceptible de limiter son utilisation contrôlée en clinique humaine.

Les conclusions toxicologiques sont les mêmes dans le cas d'une application dermique par badigeonnage pendant 42 jours consécutifs d'une solution huileuse de concentration constante de 30 ppm du nouveau médicament, à raison de 2 fois par jour. Les seuls effets irritatifs cutanés que l'on a pu constater correspondaient à l'action normale attendue.

On a étudié l'activité pharmacologique du 5-méthoxy-psoralène chez les malades atteints de psoriasis et constaté que ce produit administré par voie orale ou par voie topique, et activé par irradiation aux ultra-violets A, entraînait la disparition des plaques de psoriasis et conduisait à un "blanchiment" remarquable.

Dans l'administration par voie orale les doses utilisables par séance vont de 40 mg à 300 mg de 5-méthoxy-psoralène, et se situent généralement entre 60 et 150 mg. La dose choisie est naturellement toujours fonction de la sensibilité particulière du sujet traité.

Le nouveau médicament peut se présenter sous différentes formes telles que des comprimés, des gélules, etc.

Dans la réalisation de comprimés, on utilise des excipients convenables à base, par exemple de lactose et d'Encompress.

L'exemple suivant de réalisation d'un comprimé dosé à 20 mg de 5-méthoxy-psoralène est donné à titre illustratif et non limitatif.

Composition d'un comprimé de poids total 0,350 g,

| | |
|---|---|
| 5-méthoxy-psoralène | 0,020 g |
| Encompress | 0,159 g |
| Lactose | 0,159 g |
| Explotab | 0,0104 g |
| Stéarate de Magnésium | 0,0016 g |

Les comprimés peuvent naturellement contenir plus de 20 mg de 5-méthoxy-psoralène, et jusqu'à 100 mg.

Parmi les nombreuses expérimentations cliniques effectuées, on a choisi les cas suivants:

Cas n° 1.

Un malade atteint d'un psoriasis sur 50% de la surface corporelle est traité avec le nouveau médicament sous la forme de comprimés dosés à 20 mg.

Il reçoit 3 comprimés, soit 60 mg de 5-méthoxy-psoralène deux heures environ avant la séance d'irradiation aux ultra-violets A. Au début du traitement, on utilisait une énergie de 3 joules/cm² pour atteindre 10 joules/cm² en fin de traitement.

Les séances ont lieu 4 fois par semaine.

On obtient au bout de 30 séances un "blanchiment", c'est-à-dire une disparition des taches, à 95%.

La tolérance est excellente, pas de nausées ni maux de tête.

Cas n° 2.

Une malade atteinte de psoriasis est traitée au moyen du nouveau médicament sous forme de comprimés dosés à 40 mg de composé actif.

Elle absorbe 2 comprimés soit 80 mg de 5-méthoxy-psoralène 2 heures avant une irradiation aux ultra-violets A. L'irradiation va progressivement de 4 joules/cm² à 10 joules/cm² en fin de traitement. Les séances ont lieu 4 fois par semaine.

Au bout de 26 séances, on obtient un "blanchiment" à 95% avec une tolérance parfaite, sans nausée et sans érythème.

Cas n° 3.

Un malade atteint de psoriasis reçoit deux comprimés dosés à 20 mg, soit 40 mg de 5-méthoxy-psoralène 2 heures avant une séance d'irradiation aux ultra-violets A. L'irradiation va progressivement de 1,5 joule/cm² à 9 joules/cm² en fin de traitement.

Les séances ont lieu 4 fois par semaine.

On obtient un résultat positif à plus de 95% au bout de 19 séances, et il n'y a pas de récidive après 5 mois de recul.

La tolérance est excellente.

Cas n° 4.

Une malade atteinte de psoriasis reçoit 5 comprimés dosés à 20 mg, soit 100 mg de 5-méthoxy-psoralène 2 heures avant une séance d'irradiation aux ultra-violets A. L'irradiation va progressivement de 1 à 8 joules/cm$^2$ en fin de traitement.

Les séances ont lieu 4 fois par semaine.

On obtient un blanchiment à plus de 95% au bout de la 12ème séance. La tolérance est excellente.

L'administration du 5-méthoxy-psoralène par voie topique dans le traitement du psoriasis peut se faire sous différentes formes, telles que des solutions huileuses, des émulsions aqueuses, des pommades.

D'une manière générale la concentration en 5-méthoxy-psoralène de ces formes pour administration topique se situe entre 100 ppm et 1000 ppm. L'irradiation aux ultra-violets A est pratiquée environ 2 heures après.

On donne ci-après à titre illustratif quelques exemples d'excipients convenables, c'est-à-dire assurant une bonne solubilité du produit actif, une bonne pénétration cutanée et une bonne stabilité de la préparation.

Exemple d'excipient huileux.

| | |
|---|---|
| Huile de germe de blé | 1,0 g |
| Myristate d'isopropyle | 24,9 g |
| Butyl-hydroxy-toluène | 0,1 g |
| Huile d'arachide q.s.p. | 100,0 g |

Exemple d'emulsion aqueux.

| | |
|---|---|
| 1,2 propylène glycol | 70 g |
| Benzalkonium | 2 g |
| Alcool cetylique (Cetiol V) | 24 g |
| Cire de Lanette O | 16 g |
| Blanc de baleine | 8 g |
| Eau distillée q.s.p. | 1000 g |

Exemple d'excipient pour une pommade.

| | |
|---|---|
| Lauryl sulfate de sodium | 1,00 g |
| Propylène glycol | 6,00 g |
| Alcool stéarylique | 10,00 g |
| Vaseline | 46,00 g |
| Para-hydroxybenzoate de méthyle | 0,05 g |
| Eau purifiée q.s.p. | 100,00 g |

Dans les concentrations indiquées précédemment de 100 à 1000 ppm, le 5-méthoxy-psoralène donne par voie topique de bons résultats dans le traitement du psoriasis mais avec une rapidité d'action parfois inférieure à celle obtenue dans les traitements par voie orale.

Le traitement topique est particulièrement recommandé dans les cas de psoriasis localisés peu étendus, notamment aux articulations. Le traitement topique a une efficacité remarquable sur les plaques rebelles pouvant subsister après un traitement par voie orale.

A partie d'une concentration de 300 ppm, on a un blanchiment à 100%, avec disparition totale des plaques rebelles.

L'efficacité est la même, quelle que soit la préparation galénique choisie. Le malade quant à lui semble toujours préférer l'huile. La tolérance est très bonne mise à part quelques rares érythèmes bénins.

A titre illustratif, on peut citer les expérimentations suivantes:

Cas n° 1.

Un malade atteint de psoriasis est traité par du 5-méthoxy-psoralène par voie orale à raison de 50 mg par séance et une irradiation ultra-violets A d'énergie allant de 1,5 à 5,5 joules/cm$^2$ présente au bout de la 25ème séance une amélioration à 85% et la persistance de plaques rebelles.

On lui administre alors des applications topiques de solution huileuse à 200 ppm de 5-méthoxy-psoralène avant la séance d'irradiation et on constate une disparition totale des plaques au bout de 10 séances. Le blanchiment est donc de 100% avec une tolérance parfaite.

Cas n° 2.

Un malade se trouvant dans une situation analogue à celle du cas précédent, c'est-à-dire présentant des plaques rebelles après 29 séances d'irradiation sur administration orale de 40 mg de 5-méthoxy-psoralène et une amélioration de 90%, est traité par des applications topiques d'émulsions à 300 ppm de 5-méthoxy-psoralène avant la séance d'irradiation en ultra-violets A d'énergie de 7 joules/cm$^2$. On constate un blanchiment à 100% au bout de la 7ème séance.

La tolérance est bonne et il n'y a pas de récidive après 6 mois de recul.

Cas n° 3.

Un enfant atteint de psoriasis commence un traitement avec 40 mg par séance de 5-méthoxy-psoralène sous forme de comprimés. A partir de la dixième séance on ajoute une application topique d'émulsion à 400 ppm de 5-méthoxy-psoralène. L'irradiation ultra-violets A avait une énergie allant de 2 à 8 joules/cm$^2$. Le blanchiment est obtenu au bout de la dixième séance de traitement combiné et la tolérance fut excellente.

On peut également utiliser le 5-méthoxy-psoralène sous forme de bains dans des concentrations de l'ordre de 1500 mg pour 150 litres d'eau chaude. Le bain dure 20 minutes et est immédiatement suivi de la séance d'irradiation aux ultra-violets A. Ces séances à raison de 2 fois par semaine donnent d'excellents résultats.

Il est à remarquer en ce qui concerne l'intensité d'irradiation que la faible toxicité du 5-méthoxy-psoralène et l'importance accrue corrélative des doses utilisables permet de réduire l'énergie utile des ultra-violets A. Ceci a pour effet de diminuer les éventuels effets toxiques à long terme des irradiations répétées.

Il y a lieu de préciser que le 5-méthoxy-psoralène est également utile et efficace dans le traitement de dermatoses diverses, du vitiligo, de l'eczéma atypique et du mycosis fongoïde.

**Revendications**

1. Procédé de synthèse du 5-méthoxy-psoralène de formule:

OCH$_3$

5

O O O

caractérisé en ce que l'on utilise comme produit de départ le phloroglucinol de formule:

OH

HO OH

— on procède à une méthylation du phloroglucinol par le méthanol on présence d'un courant d'acide chlorhydrique gazeux qui donne un mélange d'éthers mono et diméthylique du phoroglucinol, dont on isole l'éther monométhylique par distillation fractionnée,

— on procède à la cyclisation de l'éther monométhylique du phloroglucinol avec du chloro acéto-nitrile,

du chlorure de zinc, un courant d'acide chlorhydrique gazeux et de l'acétate de potassium pour obtenir l'hydroxy-6-méthoxy-4-coumaranone-3 de formule:

— on procède à la réduction de cette dernière avec de l'hydrate d'hydrazine et de la potasse pour obtenir l'hydroxy-6-méthoxy-4 coumarane de formule:

— on procède à une nouvelle cyclisation avec de l'acrylonitrile du chlorure de zinc et de l'acide chlorhydrique gazeux pour obtenir le tétrahydro-3, 4, 4', 5(méthoxy-5-psoralène de formule:

— on procède enfin à la déshydrogénation de ce dernier pour obtenir le 5-méthoxy-psoralène.

2. Procédé de synthèse du 5-méthoxy-psoralène selon la revendication 1, caractérisé en ce que la réduction de l'hydroxy-6-méthoxy-4-coumarane-3-one en hydroxy-6-méthoxy-4-coumarane se fait en la dissolvant à chaud dans du diéthylène-glycol, on ajoutant de l'hydrate d'hydrazine à 98%, en chauffant à reflux pendant 15 mn, en ajoutant après refroidissement, de la potasse en pastilles et en portant à douce ébullition pendant une dizaine d'heures.

3. Nouveau médicament pour le traitement des dermatoses et en particulier du psoriasis, caractérisé en ce qu'il contient comme substance active du 5-méthoxy-psoralène sous forme de comprimés ou de gélules dosés de 20 mg à 100 mg en 5-méthoxy-psoralène.

4. Nouveau médicament pour le traitement des dermatoses et en particulier du psoriasis caractérisé en ce qu'il contient comme substance active du 5-méthoxy-psoralène sous forme de solutions huileuses, d'émulsions aqueuses, ou de pommades dans des concentrations en 5-méthoxy-psoralène allant de 100 ppm à 1000 ppm.

5. Nouveau médicament pour le traitement des dermatoses et en particulier du psoriasis caractérisé en ce qu'il contient comme substance active du 5-méthoxy-psoralène sous forme de bains dans des concentrations de l'ordre de 1500 mg de 5-méthoxy-psoralène pour 150 litres d'eau.

**Patentansprüche**

1. Verfahren zum Synthetisieren von 5-Methoxypsoralen der Formel

dadurch gekennzeichnet, daß als Ausgangsprodukt Phloroglucin der Formel

eingesetzt wird,
daß das Phloroglucin dann in Gegenwart eines Stromes von gasförmigem Chlorwasserstoff mit Methanol methyliert wird, wobei eine Mischung von Phloroglucinmonomethyläther und Phloroglucindimethyläther gebildet wird, aus der der Monomethyläther durch fraktionierte Destillation isoliert wird,
daß der Phloroglucinmonomethyläther dann mit Chloracetonitril, Zinkchlorid, einem Strom von gasförmigem Chlorwasserstoff und Kaliumacetat cyclisiert wird, wodurch 6-Hydroxy-4-methoxycumaran-3-on der Formel:

erhalten wird,
daß das 6-Hydroxy-4-methoxycumaran-3-on dann mit Hydrazinhydrat und Kaliumhydroxid reduziert wird, wodurch 6-Hydroxy-4-methoxycumaran der Formel:

erhalten wird,
daß dann eine weitere Cyclisierung mit Acrylnitril, Zinkchlorid und gasförmigem Chlorwasserstoff durchgeführt wird, wodurch 3,4,4',5'-Tetrahydro-5-methoxypsoralen der Formel:

erhalten wird, und

daß das 3,4,4',5'-Tetrahydro-5methoxypsoralen schließlich dehydriert wird, wodurch 5-Methoxypsoralen

erhalten wird.

2. Verfahren zum Synthetisieren von 5-Methoxypsoralen nach Anspruch 1, dadurch gekennzeichnet, daß die Reduktion von 6-Hydroxy-4-methoxycumaran-3-on zu 6-Hydroxy-4-methoxycumaran durchgeführt wird, indem das 6-Hydroxy-4-methoxycumaran-3-on in der Wärme in Diäthylenglykol aufgelöst wird, indem 98 %-iges Hydrazinhydrat hinzugegeben wird, indem 15 min lang unter Rückfluß erhitzt wird, indem nach dem Abkühlen Kaliumhydroxid in Form von Plätzchen hinzugegeben wird und indem etwa 10 h lang schwach sieden gelassen wird.

3. Arzneimittel zur Behandlung von Hautkrankheiten und insbesondere von Psoriasis, dadurch gekennzeichnet, daß es als aktive Substanz 5-Methoxypsoralen in Form von Tabletten oder Gelatinekapseln mit einer 5-Methoxypsoralen-Dosis von 20 mg bis 100 mg enthält.

4. Arzneimittel zur Behandlung von Hautkrankheiten und insbesondere von Psoriasis, dadurch gekennzeichnet, daß es als aktive Substanz 5-Methoxypsoralen in Form von öligen Lösungen, wäßrigen Emulsionen oder Pomaden bzw. Salben, in denen die Konzentration des 5-Methoxypsoralens 100 ppm bis 1000 ppm beträgt, enthält.

5. Arzneimittel zur Behandlung von Hautkrankheiten und insbesondere von Psoriasis, dadurch gekennzeichnet, daß es als aktive Substanz 5-Methoxypsoralen in Form von Bädern, in denen die Konzentration des 5-Methoxypsoralens in der Größenordnung von 1500 mg pro 150 Liter Wasser liegt, enthält.

## Claims

1. A synthesis method of producing methoxy-5-psoralene of formula:

comprising using as starting product phloroglucinol of formula:

— carrying out a methylation of the phloroglucinol by methanol in presence of a gaseous hydrochloric acid stream providing a mixture of mono and dimethyl esters of the phloroglucinol, the monomethyl ether of which is isolated by fractional distillation,

— carrying out the cyclisation of the phloroglucinol monométhyl ether with chloroacetonitrile, zinc chloride, a gaseous hydrochloric acid stream and potassium acetate for obtaining hydroxy-6-methoxy-4-coumaranone-3 of formula:

— carrying out the reduction of the latter with hydrazine hydrate and potassium carbonate for obtaining hydroxy-6-methoxy-4-coumarane of formula:

— carrying out a new cyclisation with acrylonitrile, zinc chloride and gaseous hydrochloric acid for obtaining tetrahydro-3, 4, 4′, 5(methoxy-5-psoralene) of formula:

— carrying out finally the dehydrogenation of the latter for obtaining methoxy-5-psoralene:

2. A synthesis method of producing methoxy-5-psoralene according to claim 1, wherein the reduction of hydroxy-6-methoxy-4-coumarane-3-one into hydroxy-6-methoxy-4-coumarane is carried out by dissolving it when hot in diethylene glycol, by adding hydrazine hydrate at 98%, by reflux heating for 15 mn, by adding after cooling down potassium hydroxyde in lozenges and by boiling gently for about ten hours.

3. A new drug for the treatment of dermatoses and particularly psoriasis, characterized in that it contains as active substance 5-methoxy-psoralene presented in the form of tablets or gelules containing from 20 to 100 mg of methoxy-5-psoralene.

4. A new drug for the treatment of dermatoses and particularly psoriasis, characterized in that it contains as active substance 5-methoxy-psoralene presented as oily solutions, aqueous emulsions or ointments having concentrations of methoxy-5-psoralene from 100 to 1000 ppm.

5. A new drug for the treatment of dermatoses and particularly psoriasis, characterized in that it contains as active substance 5-methoxy-psoralene presented as baths having concentrations of the order of 1500 mg of methoxy-5-psoralene for 150 litres of water.